# EUROPEAN PATENT SPECIFICATION

(11) **EP 3 803 820 B1**
(45) Date of publication and mention of the grant of the patent: **30.10.2024**
(21) Application number: 19732183.9
(22) Date of filing: 03.06.2019
(51) Int. Cl.: G08B 21/04, G08B 13/00

(54) **TRACKING INDIVIDUAL USER HEALTH USING INTRUSION DETECTION SENSORS**
VERFOLGUNG VON INDIVIDUELLER BENUTZERGESUNDHEIT MITTELS INTRUSIONSDETEKTIONSSENSOREN
SUIVI DE LA SANTÉ D'UN UTILISATEUR INDIVIDUEL À L'AIDE DE CAPTEURS DE DÉTECTION D'INTRUSION

(30) Priority: 05.06.2018 IN 201811021043
(43) Date of publication of application: 14.04.2021
(73) Proprietor: Carrier Corporation, Palm Beach Gardens, FL 33418 (US)
(72) Inventor: NALLAPERUMAL, Pirammanayagam, Madhapur, Hyderabad, Telangana 500081 (IN); GAJULA, Saikrishna, Madhapur, Hyderabad, Telangana 500081 (IN); RAMOUTAR, Michael, Lincolnton, NC 28092 (US)
(74) Representative: Dehns
(86) International application number: PCT/US2019/035111
(87) International publication number: WO 2019/236440

(56) References cited:
- US-A1- 2016 328 941
- US-A1- 2017 018 159
- US-A1- 2017 098 367

## Description

### BACKGROUND

The present disclosure generally relates to a method and system for tracking health, and more particularly to tracking user health using intrusion detection sensors.

Intrusion detection systems are used to secure access to various buildings and structures. For example, the entrances and exits of a building can be secured with keyed access and common areas may be monitored with motion detectors and cameras. Also, intrusion detection systems include various components including both hardware and software components that function together to monitor the desired areas. The hardware components can include equipment for monitoring and detecting motion and sound, capturing video. Additionally, other types of sensors can be used to detect conditions to secure the premises. Software components can be used to provide real-time services to a mobile application or to a monitoring service. Users, through the mobile applications, are provided the ability to interface and control the systems from a remote location such as switching modes of the system from a disarmed mode to an armed mode. Intrusion detection systems provide the comfort and convenience to users by securing one's premises.

US 2016/0328941 A1 relates to a system and method for observing the presence, location, movement, and/or attitude of one or more objects to be monitored. In a first mode the system checks the attitude and/or state of health of the object. In a second mode the system functions as an intruder alarm.

### BRIEF DESCRIPTION

According to a first aspect of the invention, a system for tracking user health using intrusion detection sensors is provided. The system includes a processor module, one or more sensors of an intrusion detection system configured to measure one or more metrics of users, the one or more sensors are configured to operate in a first mode and a second mode, wherein the processor module is operably coupled to the one or more sensors, and one or more devices operably coupled to the processor module to identify the users. The system also includes a health profile having a user profile including one or more metrics from the one or more sensors and user identifiers from the one or more devices, and a transmitter coupled to the processor module configured to transmit notification based at least in part on a detection associated with the one or more metrics. The system includes a processor that is configured to detect the one or more metrics in a first mode and detect a presence of an intruder in a second mode. The first mode is an unarmed mode and that the second mode is an armed mode. The one or more sensors include a radar based sensor that is configured to measure a user heartbeat and/or respiration rate. The radar based sensor detects the one or more metrics and an intrusion. The one or more metrics of the health profile includes respiration rate and/or heartbeat rate information of the users.

The one or more sensors of the system may include a camera that is configured to identify the user based on facial recognition.

The system may include identifying the users by entering a PIN code into a control panel of the intrusion detection system to arm/disarm the intrusion detection system.

The system may include at least one of wearable devices that are configured to measure one or more metrics and confirm the one or more metrics the users obtained or an indoor positioning system configured to distinguish the one or more metrics received by the radar based sensor.

The system may include transmitting, by the transmitter, the notification based at least in part on receiving a request from a user.

According to a second aspect of the invention, a method for tracking user health using intrusion detection sensors is provided. The method includes receiving user data of a user from one or more devices, receiving one or more metrics from one or more sensors of an intrusion detection system, and mapping, in a health profile, the user data with the sensor data. The method also includes determining a condition of a user based at least in part on the health profile, and transmitting a notification based at least in part on the determined condition. The method includes detecting the one or more metrics in a first mode and detecting a presence of an intruder in a second mode. The method includes a first mode that is an unarmed mode of the intrusion detection system and the second mode that is an armed mode. The one or more sensors include a radar based sensor that is configured to measure a user heartbeat and/or respiration rate. The radar based sensor detects the one or more metrics and an intrusion. The one or more metrics of the health profile includes respiration rate and/or heartbeat rate information of users.

The one or more sensors of the method may include a camera that is configured to identify the user based on facial recognition.

The method may include identifying users while entering a PIN code into a control panel of the intrusion detection system to arm/disarm the intrusion detection system.

The method may include at least one of wearable devices that are configured to measure one or more metrics and confirm the one or more metrics the users obtained or an indoor positioning system configured to distinguish the one or more metrics received by the radar based sensor.

The method may include transmitting the notification based at least in part on receiving a request from a user.

### BRIEF DESCRIPTION OF THE DRAWINGS

The following descriptions should not be considered limiting in any way. With reference to the accompanying drawings, like elements are numbered alike:
FIG. 1 depicts a system for tracking user health using intrusion system sensors in accordance with one or more embodiments;
FIG. 2 depicts a health profile for tracking user health using intrusion system sensors in accordance with one or more embodiments; and
FIG. 3 depicts a flow chart of a method for tracking user health using intrusion system sensors in accordance with one or more embodiments.

### DETAILED DESCRIPTION

Cardiovascular disease is among the leading health concerns in the world. Many forms of diagnosis and treatment include monitoring the heart, blood pressure, and respiration rate. The monitoring can be performed using different techniques and devices which include electrocardiograms, computed tomography (CT) and magnetic resonance imaging (MRI). In addition, the monitoring of a person's heartbeat and/or respiration rate can be measured using sensors that are based on radar technology.

In today's environment, security systems focus on intrusion detection of a user's home or premises where some systems incorporate the use of the radar type sensors mentioned above for motion detection. Security systems are also equipped with a variety of other sensor types such as vibration detectors, proximity sensors, lights curtains, hazardous gas detectors, just to name a few. These sensors can be positioned in strategic locations to ensure the desired areas/zones are being monitored. The security systems perform the monitoring and detection when in the armed state.

However, one or more embodiments leverage the use of existing sensors in the security system to obtain health related information of those present in the monitored area when the system is in the disarmed mode. In addition, a user health profile can be created for each user and track any deviations from the normal conditions measured by the sensors. The conditions include monitoring the heartbeat and/or respiration rate of the users. The measured conditions are mapped to the users based on user interaction with the intrusion detection system, such as using a PIN code to disarm the device, location information from user devices, connection information from user devices, facial recognition from image capturing devices, etc.

In one or more embodiments, health profiles can be generated for a plurality of users and stored to track the health related information obtained by the security system. The continuous tracking of health related information over a period of time can reveal any abnormalities in the health of its users by analyzing patterns of measurements or sharp deviations from normal (average) data corresponding to the measured health parameters. The security system is also configured to trigger an alert to appropriate authorities, emergency responders, and contacts upon the detection of an abnormal event. The health profile can also store information obtained from other wearable devices which can provide additional metrics for analysis or confirm data collected by other sensors/devices that correspond to information of a user's health profile.

The security system is operated in a plurality of modes including an armed mode and unarmed mode. During a first mode the security system is configured to perform the intrusion detection monitoring the premises and in the unarmed mode the security system is configured to perform the health monitoring.

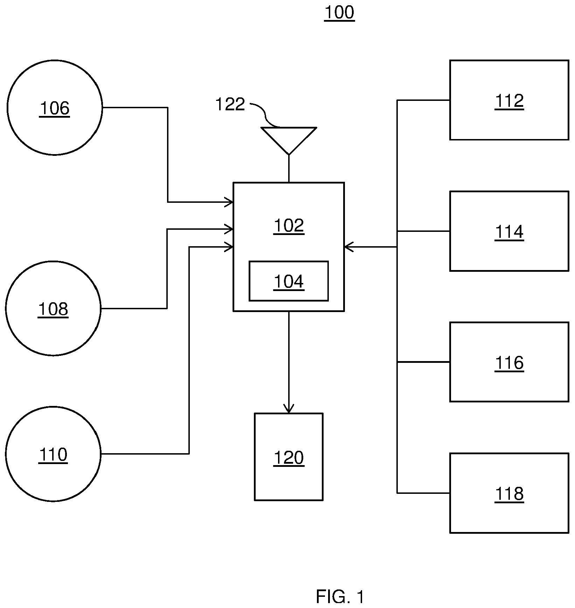

In FIG. 1, a system for tracking individual user health using intrusion detection system sensors is shown. The intrusion detection system 100, hereinafter referred to as the system 100, includes a processor 102 where the processor 102 includes a processor, memory, and appropriate interfaces (not shown) and is configured to communicate over a network. In addition, the processor 102 is further configured to communicate to an external network. The processor 102 can be configured to communicate over wired and/or wired connections such as Ethernet and Wi-Fi.

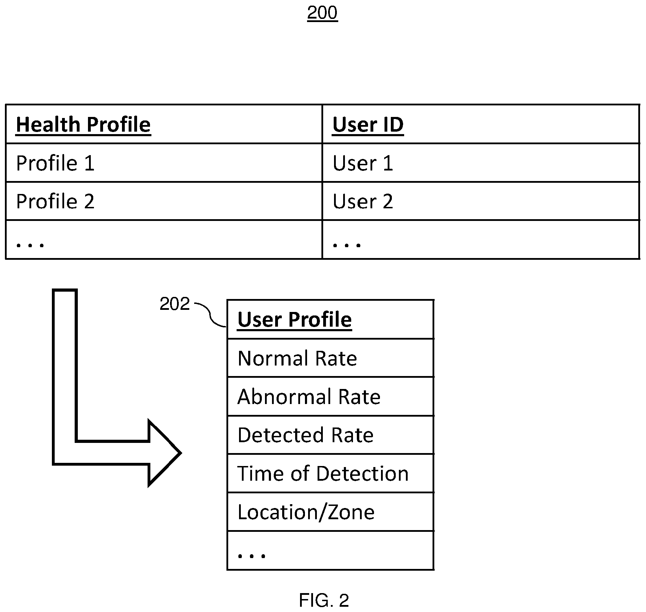

As shown in FIG. 1, the processor 102 includes a health profile 104 (described in more detail with reference to FIG. 2). It should also be understood the health profile 104 can be stored in a different location of the system 100 or externally in a database connected over a network.

FIG. 1 as shown provides a plurality of sensors that are coupled to the processor 102. Security and intrusion detection systems include a variety of sensors including motion detectors, vibration sensors, cameras, etc. to monitor the premises. Generally, the sensors are not in the monitoring state when in the unarmed mode and are in the active monitoring state when in the armed mode. Systems include a radar type of sensor to detect motion of an intruder. In one or more embodiments, the techniques described herein leverage the existing sensors of the system 100 to track health related data when the system 100 is not in the armed mode. According to the invention, the system 100 is configured to collect data related to the heartbeat and/or respiration rate for one or more user(s) when in the disarmed mode using a sensor based on radar technology.

Current radar technology enables the measurement of respiration/heartbeat of users in the vicinity. Radar provides electromagnetic radiation towards the body of the human within its proximity where the movement of the body surface, such as the chest are, is used to measure a result. The measurements, using the electromagnetic radiation, can be used to extract diagnostic data about the user. The data can be converted to a form that is easily comprehendible to a person such as a heart rate value or graph form. In one or more embodiments, the collected data can be correlated to blood pressure or other types of cardiovascular information and vital sign data. The radar detection device can be configured to determine the heart rate and respiration rate of a person that is both stationary and mobile.

The system 100 uses the radar type sensor for intrusion detection to function as a heartbeat detector 106 as shown in FIG. 1. The heartbeat detector 106 is configured to emit an electromagnetic signal towards an object or person and measure the reflection received. Based on the received signal the heartbeat/respiration rate of a person can be determined and stored in the memory of the processor 102 or other location. In one or more embodiments, the configuration of the heartbeat detectors 106 such as electromagnetic radiation strength, frequency, etc. can be adapted to the environment as needed.

In addition, the system 100 can receive data from other sensors such wearable technology 108 including smart watches and fitness/health trackers. These devices are equipped with heart monitoring sensors which, if coupled to the system 100, the processor 102 can use the data to detect and/or compare the heart rate with data collected by the heartbeat detector 106. Other sensors 110 can be coupled to the system 100 to collect additional information such as temperature data which can indicate if the temperature in the location is unusually warm/cold, smart devices/appliances which can include speakers that indicate the location of the user based on detecting sounds of the user, sensors indicating the opening/closing of a door which can indicate the last position of a user, etc.

In FIG. 1, the system 100 includes a control panel 112 which can be used to change the operational modes of the system 100. The control panel 112 can include a display indicating the status of the system 100 and keys to input data such as a PIN code. In one or more embodiments, the PIN code of a particular user can be entered when arming/disarming the system 100, which can identify a particular user that is entering the home. The PIN code can be mapped to the data that is collected by the system 100 from the user.

The system 100 can include cameras 114 to monitor various locations of the premises. For example, cameras 114 can be used to monitor the entrances and exits of the premises or the cameras 114 can be used to monitor zones that are of particular interest within the premises. In one or more embodiments, the images captured from the cameras 114 can identify the user monitor by using facial recognition techniques. The location of the cameras 114 can be used to provide an indication of the location of the identified user that is being monitored by the heartbeat detector 106 or other sensors.

The system 100 can also leverage the indoor positioning system 116 which can be included as part of a mobile phones/tablet/computer or wearable technology. The system 100 can collect this data to identify the location of the user and map the monitored health conditions to that user. The indoor positioning system 116 can be used to map a particular user among a plurality of users present within range of the sensors that are performing the health related measurements. In a given scenario, multiple users may be present in a sensor zone where the sensor possibly receives multiples sets of metrics at a time. The system 100 can distinguish and map the metrics to the appropriate health/user profiles using the indoor based positioning system 116 e.g., beacon based systems.

The location and identify of the user(s) can also be determined by the devices that the user has that are connected to a known network such as a Wi-Fi network. The identifier of the device can be mapped to a user when monitoring the health related data. In addition, the signal strength of the connected device to an access point can be used to approximate the location of the user. In one or more embodiments the location information based on the signal strength to an access point can be confirmed by using the location data received by the heartbeat sensors 106, indoor positioning system 116, cameras 114, etc.

As described above, the location of the user can be determined based on a number of techniques. The radar technology can be used to determine the distance the user is from the sensor. Location information can be gathered according to indoor positioning system information provided by an electronic device such as a smart phone and/or smartwatch. In addition, a connection address and signal strength of an access point can be used to determine

In one or more embodiments, additional sensors can include data from smartwatches, heart rate monitors, smart appliances, smart TV's, etc. The smart appliances generally indicate when a device has been opened/closed or powered on/off which can provide an indication as to the location of the user.

In other embodiments, a plurality of heartbeat detectors 106 can be configured throughout the home and used to detect a plurality of users and locations/zones of those users.

The system 100 is configured to detect an abnormal state for respective users to trigger an alert to various contacts and/or systems 120. In one or more embodiments, the contacts are based on a configurable contact list. In another embodiment, the systems 120 include sending an alert to a monitoring service that notifies the proper authorities or can prompt the user for further action. The system 100 is an example configuration and other arrangements of sensors, controllers, devices, etc. can be used. In one or more embodiments, the system 100 is configured to transmit the notification using a transmitter 122.

In FIG. 2, a health profile 200 used for tracking user health using intrusion detections sensors in accordance with one or more embodiments is shown. In one or more embodiments, the health profile 200 can be stored locally such as in the processor 102 as shown in FIG. 1. In other embodiments, the health profile 200 can be stored in a remote storage over a network such as in a remote database. In either scenario, the health profile 200 is assessable to the intrusion detection system to perform health analysis described herein.

The health profile 200 includes multiple user profiles 202. As shown in FIG. 2, the health profile 200 includes a first user profile "Profile 1" and a second profile "Profile 2." Each user profile is mapped/associated with a specific user identity. It should be understood that any number of user profiles 202 can be stored in the health profile 200 although only two user profiles 202 are shown. In one or more embodiments, the users can include family members, roommates, common tenants, and/or frequent visitors that are regularly present or in range of the intrusion detection system 100.

As shown in FIG. 2, a user profile 202 includes various types of data. The user profile 202 includes information related to a particular user's normal heart rate that has been measured by the system 100. This can be determined by averaging the detected heartbeat of the user over a period of time such as over a week, month, year or other configurable time period. The normal heart rate can be updated to reflect the most recent readings of the user.

The user profile 202 can also store data indicating a threshold for an abnormal heart rate to trigger an alert or alarm. The threshold for the abnormal rate can be stored as a deviation such as a ratio or percentage from the normal rate or the abnormal rate can be a specific rate or range. In the event multiple ranges are used a first range can be used to indicate an elevated heart rate to the user prior to triggering an alarm for the exceeding the threshold. Upon meeting and/or exceeding the threshold an appropriate alert can be triggered.

The user profile 202, can also store the current detected heart/respiration rate that is compared to the normal and abnormal rates. In one or more embodiments, information such as the detected heart/respiration rate, time of detection, location/zone information can be stored over a period of time to track and monitor the user's health over a period of time. Over time as more data points are collected for each user various patterns can be recognized. For example, if a user routinely has an elevated hear rate for a duration of time at the same time every day and/or week, the system 100 can infer this is a normal condition. The user may exercise or have just performed an activity elevating the heart rate that is part of their daily routine. Similarly, a reduced heart rate that is recognized at approximately the same time slot can be associated with a particular activity such as napping and/or other relaxing activity such as reading. These patterns can be learned for each user over a period of time and used to determine whether to transmit an alert when comparing the current heart rate to the threshold data. Other contextual data can be used to determine whether or not to trigger an alert. For example, in the event a sudden shift in heart rate that is uncharacteristic to a particular user is detected, an alert can be triggered.

In addition, other information can be stored in the user profile 202 such as a configurable emergency contact list, last abnormal event data, temperature data, etc. The health profile 200 and user profile 202 provide a non-limiting illustration of the collected data and is not meant to be limiting as to the scope and type of information that can be stored for the users.

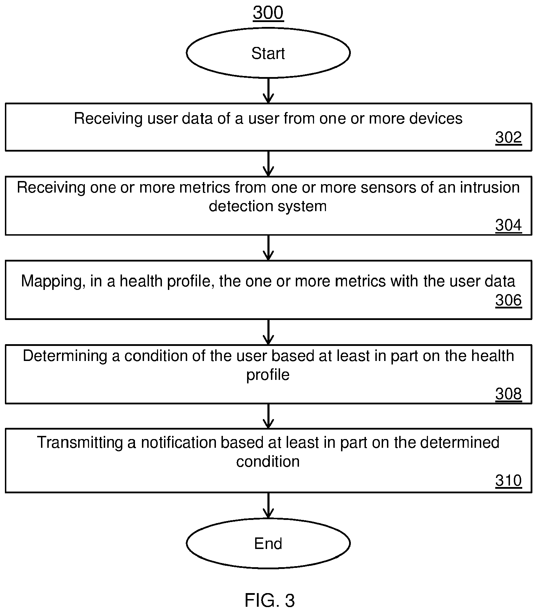

Now referring to FIG. 3, a flow chart of a method 300 for tracking individual user health using intrusion detection sensors in accordance with one or more embodiments is shown.

The method 300 begins and proceeds to block 302 which provides for receiving user data for a user from one or more devices. The user data includes user identity information which can be determined by a plurality of techniques. For example, a user identity can be determined by a personal PIN code used to disarm the mode of the system at a control panel. In another example, a camera that is coupled to the security system can be used to identify a user to be monitored. Other techniques include determining a user device that is connected to a network such as a Wi-Fi network, Bluetooth network, or other type of network can be used to identify the user.

At block 304, the method 300 provides for receiving one or more metrics from one or more sensors of an intrusion detection system. In one or more embodiments, the radar type sensor collects data by emitting an electromagnetic signal towards a user to obtain respiration and heartbeat information. In embodiments, other sensors such as wearable devices and smart appliances can be used.

Block 306 provides for mapping, in a health profile, the one or more metrics with the user data. Responsive to identifying the user and performing health related measurements for the user, the data is stored in a memory that associates the measured data to the respective user. The system can be configured to perform the health related measurements for a plurality of users. Therefore, it is critical the appropriate users are identified when storing the collected data. In addition, location information for the user can be determined by using the heartbeat detectors 106, PIN codes, cameras 114, indoor positioning system readings, connected devices 118 signal strength and unique identification number (Bluetooth ID, Wi-Fi MAC), etc.

Block 308 provides for determining a condition of the user based at least in part on the health profile. The method 300 proceeds to block 310 and provides for transmitting a notification based at least in part on the determined condition. In one or more embodiments, the transmission of the notification can be based on a configurable threshold, such receiving a number of samples of metrics. The notification can be transmitted to a configurable contact list including guardians and appropriate authorities such as emergency responders including EMT's and the fire department personal, etc. The alert information can include the user identifier information, location information, and other data that is available in the user health profile. In other embodiments, the notification can be transmitted to one or more respective users responsive to a user request. Requests can be transmitted using a device such as but not limited to a mobile device/tablet.

The technical benefits and effects include levering a security system to provide health related information in addition to providing normal home intrusion monitoring. The sensors can be effectively utilized for intrusion detection when users are away from home and perform health monitoring when users are within the premises.

A detailed description of one or more embodiments of the disclosed apparatus and method are presented herein by way of exemplification and not limitation with reference to the Figures.

The term "about" is intended to include the degree of error associated with measurement of the particular quantity based upon the equipment available at the time of filing the application.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present invention has been described with reference to an exemplary embodiment or embodiments, it will be understood by those skilled in the art that various changes may be made without departing from the scope of the present invention. In addition, many modifications may be made to adapt a particular situation or material to the teachings of the present invention without departing from the scope of the claims. Therefore, it is intended that the present invention not be limited to the particular embodiment disclosed, but that the present invention will include all embodiments falling within the scope of the claims.

## Claims

1. A system (100) for tracking individual user health using intrusion detection sensors, comprising:
a processor module (102);
one or more sensors of an intrusion detection system (100) configured to measure one or more metrics of users, wherein the one or more sensors are configured to operate in a first, unarmed mode to detect the one or more metrics and a second, armed mode to detect a presence of an intruder, wherein the processor module (102) is operably coupled to the one or more sensors;
one or more devices operably coupled to the processor module (102) to identify the users;
a health profile (104, 200) comprising user identifiers from the one or more devices; and
a transmitter (122) coupled to the processor module (102);
**characterized in that**:
the one or more sensors include a radar-based sensor, and wherein the radar-based sensor is configured to detect motion of the intruder and measure a user heartbeat and/or respiration rate;
the health profile (104, 200) comprises a user profile (202) including the user heartbeat and/or respiration rate from the one or more sensors; and
the transmitter (122) is configured to transmit a notification based at least in part on a detection associated with the user heartbeat and/or respiration rate.

2. The system of claim 1, wherein one or more sensors include a camera that is configured to identify the user based on facial recognition.

3. The system of claim 1, wherein the users are identified while entering a PIN code into a control panel (112) of the intrusion detection system (100) to arm/disarm the intrusion detection system.

4. The system of claim 1, further comprising at least one of wearable devices (108) configured to measure one or more metrics and confirm the one or more metrics the users obtained or an indoor positioning system configured to distinguish the one or more metrics received by the radar based sensor.

5. The system of claim method of claim 1, wherein the transmitter (122) transmits the notification based at least in part on receiving a request from a user.

6. A method for tracking individual user health using intrusion detection sensors, the method comprising:
receiving user data of a user from one or more devices; and
receiving one or more metrics from one or more sensors of an intrusion detection system, wherein one or more sensors are configured to measure one or more metrics of users, and the one or more sensors are configured to operate in a first, unarmed mode to detect the one or more metrics and a second, armed mode to detect a presence of an intruder,
**characterized in that**:
the one or more sensors include a radar-based sensor, and wherein the radar-based sensor is configured to detect motion of the intruder and measure a user heartbeat and/or respiration rate;
wherein the method further comprises:
mapping, in a health profile (104, 200), the user data with the user heartbeat and/or respiration rate;
determining a condition of a user based at least in part on the user heartbeat and/or respiration rate; and
transmitting a notification based at least in part on the determined condition.

7. The method of claim 6, wherein one or more sensors include a camera that is configured to identify the user based on facial recognition.

8. The method of claim 6, wherein users are identified while entering a PIN code into a control panel (112) of the intrusion detection system (100) to arm/disarm the intrusion detection system.

9. The method of claim 6, further comprising at least one of wearable devices (108) configured to measure one or more metrics and confirm the one or more metrics the users obtained or an indoor positioning system configured to distinguish the one or more metrics received by the radar based sensor.

10. The method of claim 6, wherein transmitting the notification is based at least in part on receiving a request from a user.

## Patentansprüche

1. System (100) zum Verfolgen von individueller Benutzergesundheit mittels Intrusionsdetektionssensoren, umfassend:
ein Prozessormodul (102);
einen oder mehrere Sensoren eines Intrusionsdetektionssystems (100), die zum Messen einer oder mehrerer Metriken von Benutzern konfiguriert sind, wobei der eine oder die mehreren Sensoren zum Betreiben in einem ersten nicht aktivierten Modus zum Detektieren der einen oder mehreren Metriken und in einem zweiten aktivierten Modus zum Detektieren eines Vorhandenseins eines Eindringlings konfiguriert sind, wobei das Prozessormodul (102) mit dem einen oder den mehreren Sensoren wirkgekoppelt ist;
eine oder mehrere Vorrichtungen, die zum Identifizieren der Benutzer mit dem Prozessormodul (102) wirkgekoppelt sind;
ein Gesundheitsprofil (104, 200), das Benutzeridentifikatoren von der einen oder den mehreren Vorrichtungen umfasst; und
einen Sender (122), der mit dem Prozessormodul (102) gekoppelt ist;
**dadurch gekennzeichnet, dass**:
der eine oder die mehreren Sensoren einen radarbasierten Sensor beinhalten und wobei der radarbasierte Sensor zum Detektieren von Bewegung des Eindringlings und zum Messen eines Benutzerherzschlags und/oder einer Benutzeratemfrequenz konfiguriert ist;
das Gesundheitsprofil (104, 200) ein Benutzerprofil (202) umfasst, das den Benutzerherzschlag und/oder die Benutzeratemfrequenz von dem einen oder den mehreren Sensoren umfasst; und
der Sender (122) zum Senden einer Benachrichtigung mindestens teilweise basierend auf einer dem Benutzerherzschlag und/oder der Benutzeratemfrequenz zugeordneten Detektion konfiguriert ist.

2. System nach Anspruch 1, wobei ein oder mehrere Sensoren eine Kamera beinhalten, die zum Identifizieren des Benutzers basierend auf Gesichtserkennung konfiguriert ist.

3. System nach Anspruch 1, wobei die Benutzer beim Eingeben eines PIN-Codes in ein Bedienfeld (112) des Intrusionsdetektionssystems (100) zum Aktivieren/Deaktivieren des Intrusionsdetektionssystems identifiziert werden.

4. System nach Anspruch 1, ferner umfassend mindestens eine von tragbaren Vorrichtungen (108), die zum Messen einer oder mehrerer Metriken und zum Bestätigen der einen oder mehreren Metriken, die die Benutzer erhalten haben, konfiguriert sind, oder ein Innenraumpositionierungssystem, das zum Unterscheiden der einen oder mehreren von dem radarbasierten Sensor empfangenen Metriken konfiguriert ist.

5. System nach Anspruch 1, wobei der Sender (122) die Benachrichtigung mindestens teilweise basierend auf Empfangen einer Anforderung von einem Benutzer sendet.

6. Verfahren zum Verfolgen von individueller Benutzergesundheit mittels Intrusionsdetektionssensoren, wobei das Verfahren Folgendes umfasst:
Empfangen von Benutzerdaten eines Benutzers von einer oder mehreren Vorrichtungen; und
Empfangen einer oder mehrerer Metriken von einem oder mehreren Sensoren eines Intrusionsdetektionssystems, wobei ein oder mehrere Sensoren zum Messen einer oder mehrerer Metriken von Benutzern konfiguriert sind und wobei der eine oder die mehreren Sensoren zum Betreiben in einem ersten nicht aktivierten Modus zum Detektieren der einen oder mehreren Metriken und in einem zweiten aktivierten Modus zum Detektieren eines Vorhandenseins eines Eindringlings konfiguriert sind,
**dadurch gekennzeichnet, dass**:
der eine oder die mehreren Sensoren einen radarbasierten Sensor beinhalten und wobei der radarbasierte Sensor zum Detektieren von Bewegung des Eindringlings und zum Messen eines Benutzerherzschlags und/oder einer Benutzeratemfrequenz konfiguriert ist;
wobei das Verfahren ferner Folgendes umfasst:
Abbilden der Benutzerdaten mit dem Benutzerherzschlag und/oder der Benutzeratemfrequenz in einem Gesundheitsprofil (104, 200) ;
Bestimmen eines Zustands eines Benutzers mindestens teilweise basierend auf dem Benutzerherzschlag und/oder der Benutzeratemfrequenz; und
Senden einer Benachrichtigung mindestens teilweise basierend auf dem bestimmten Zustand.

7. Verfahren nach Anspruch 6, wobei ein oder mehrere Sensoren eine Kamera beinhalten, die zum Identifizieren des Benutzers basierend auf Gesichtserkennung konfiguriert ist.

8. Verfahren nach Anspruch 6, wobei Benutzer beim Eingeben eines PIN-Codes in ein Bedienfeld (112) des Intrusionsdetektionssystems (100) zum Aktivieren/Deaktivieren des Intrusionsdetektionssystems identifiziert werden.

9. Verfahren nach Anspruch 6, ferner umfassend mindestens eine von tragbaren Vorrichtungen (108), die zum Messen einer oder mehrerer Metriken und zum Bestätigen der einen oder mehreren Metriken, die die Benutzer erhalten haben, konfiguriert sind, oder ein Innenraumpositionierungssystem, das zum Unterscheiden der einen oder mehreren von dem radarbasierten Sensor empfangenen Metriken konfiguriert ist.

10. Verfahren nach Anspruch 6, wobei das Senden der Benachrichtigung mindestens teilweise auf Empfangen einer Anforderung von einem Benutzer basiert.

## Revendications

1. Système (100) de suivi de la santé d'un utilisateur individuel à l'aide de capteurs de détection d'intrusion, comprenant :
un module de processeur (102) ;
un ou plusieurs capteurs d'un système de détection d'intrusion (100) configurés pour mesurer une ou plusieurs métriques d'utilisateurs, dans lequel le ou les capteurs sont configurés pour fonctionner dans un premier mode non armé pour détecter la ou les métriques et un second mode armé pour détecter la présence d'un intrus, dans lequel le module de processeur (102) est couplé de manière fonctionnelle au ou aux capteurs ;
un ou plusieurs dispositifs couplés de manière fonctionnelle au module de processeur (102) pour identifier les utilisateurs ;
un profil de santé (104, 200) comprenant des identifiants d'utilisateur provenant d'un ou de plusieurs dispositifs ; et un émetteur (122) couplé au module de processeur (102) ;
**caractérisé en ce que** :
le ou les capteurs incluent un capteur basé sur un radar, et dans lequel le capteur basé sur un radar est configuré pour détecter le mouvement de l'intrus et mesurer le rythme cardiaque et/ou la fréquence respiratoire d'un utilisateur ;
le profil de santé (104, 200) comprend un profil d'utilisateur (202) incluant le rythme cardiaque et/ou la fréquence respiratoire de l'utilisateur provenant d'un ou de plusieurs capteurs ; et
l'émetteur (122) est configuré pour transmettre une notification basée au moins en partie sur une détection associée au rythme cardiaque et/ou à la fréquence respiratoire de l'utilisateur.

2. Système selon la revendication 1, dans lequel un ou plusieurs capteurs incluent une caméra qui est configurée pour identifier l'utilisateur sur la base d'une reconnaissance faciale.

3. Système selon la revendication 1, dans lequel les utilisateurs sont identifiés lors de la saisie d'un code PIN dans un panneau de commande (112) du système de détection d'intrusion (100) pour armer/désarmer le système de détection d'intrusion.

4. Système selon la revendication 1, comprenant également au moins un des dispositifs portables (108) configurés pour mesurer une ou plusieurs métriques et confirmer la ou les métriques obtenues par les utilisateurs ou un système de positionnement intérieur configuré pour distinguer la ou les métriques reçues par le capteur basé sur un radar.

5. Système selon la revendication 1, dans lequel l'émetteur (122) transmet la notification sur la base, au moins en partie, de la réception d'une demande d'un utilisateur.

6. Procédé de suivi de la santé d'un utilisateur individuel à l'aide de capteurs de détection d'intrusion, le procédé comprenant :
la réception des données utilisateur d'un utilisateur à partir d'un ou de plusieurs dispositifs ; et
la réception d'une ou de plusieurs métriques provenant d'un ou de plusieurs capteurs d'un système de détection d'intrusion, dans lequel un ou plusieurs capteurs sont configurés pour mesurer une ou plusieurs métriques d'utilisateurs, et le ou les capteurs sont configurés pour fonctionner dans un premier mode non armé pour détecter la ou les métriques et un second mode armé pour détecter la présence d'un intrus,
**caractérisé en ce que** :
le ou les capteurs incluent un capteur basé sur un radar, et dans lequel le capteur basé sur un radar est configuré pour détecter le mouvement de l'intrus et mesurer le rythme cardiaque et/ou la fréquence respiratoire d'un utilisateur ;
dans lequel le procédé comprend également :
la mise en correspondance, dans un profil de santé (104, 200), des données de l'utilisateur avec le rythme cardiaque et/ou la fréquence respiratoire de l'utilisateur ;
la détermination d'un état d'un utilisateur basé au moins en partie sur le rythme cardiaque et/ou la fréquence respiratoire de l'utilisateur ; et
la transmission d'une notification basée au moins en partie sur la condition déterminée.

7. Procédé selon la revendication 6, dans lequel un ou plusieurs capteurs incluent une caméra qui est configurée pour identifier l'utilisateur sur la base d'une reconnaissance faciale.

8. Système selon la revendication 6, dans lequel les utilisateurs sont identifiés lors de la saisie d'un code PIN dans un panneau de commande (112) du système de détection d'intrusion (100) pour armer/désarmer le système de détection d'intrusion.

9. Procédé selon la revendication 6, comprenant également au moins un des dispositifs portables (108) configurés pour mesurer une ou plusieurs métriques et confirmer la ou les métriques obtenues par les utilisateurs ou un système de positionnement intérieur configuré pour distinguer la ou les métriques reçues par le capteur basé sur un radar.

10. Procédé selon la revendication 6, dans lequel la transmission de la notification est basée au moins en partie sur la réception d'une demande d'un utilisateur.
